Europäisches Patentamt
European Patent Office
Office européen des brevets

(19)

(11) Publication number: 0 200 524 A2

(12) EUROPEAN PATENT APPLICATION

(21) Application number: 86303225.6

(22) Date of filing: 29.04.86

(51) Int. Cl.[4]: C 07 D 211/90
A 61 K 31/44

(30) Priority: 03.05.85 GB 8511350
21.01.86 GB 8601382

(43) Date of publication of application:
05.11.86 Bulletin 86/45

(84) Designated Contracting States:
AT BE CH DE FR GB IT LI LU NL SE

(71) Applicant: Pfizer Limited
Ramsgate Road
Sandwich Kent CT13 9NJ(GB)

(84) Designated Contracting States:
GB

(71) Applicant: Pfizer Corporation
Calle 15 1/2 Avenida Santa Isabel
Colon(PA)

(84) Designated Contracting States:
BE CH DE FR IT LI LU NL SE AT

(72) Inventor: Campbell, Simon Fraser, Dr.
Grey Friars Upper Street
Deal Kent(GB)

(72) Inventor: Alker, David, Dr.
1, Clive Road
Margate Kent(GB)

(74) Representative: Wood, David John et al,
Pfizer Limited Ramsgate Road
Sandwich Kent CT13 9NJ(GB)

(54) **Dihydropyridine calcium antagonists.**

(57) Dihydropyridine calcium antagonists, useful in the treatment of conditions such as angina and hypertension, of the formula:-

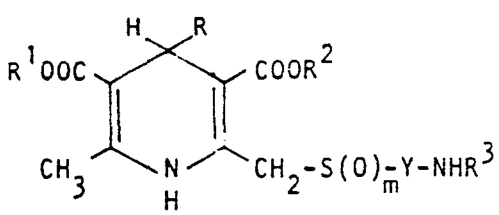

---- (I)

where

R is an optionally substituted aryl or heteroaryl group;
$R^1$ and $R^2$ are each independently selected from
i) $C_1$-$C_4$ alkyl and
ii) $C_1$-$C_4$ alkyl substituted by $C_3$-$C_7$ cycloalkyl, aryl, $CF_3$, $C_2$-$C_4$ alkanoyl, halo, hydroxy, $C_1$-$C_4$ alkoxy, $C_2$-$C_4$ alkanoyloxy, aryl-($C_1$-$C_4$ alkoxy), or $-NR^4R^5$;
m is 0, 1 or 2;
$R^3$ is H, $C_1$-$C_4$ alkyl or $-COCF_3$;
$R^4$ is H or $C_1$-$C_4$ alkyl;
$R^5$ is H, $C_1$-$C_4$ alkyl or aryl-($C_1$-$C_4$ alkyl);
and
Y is $-(CH_2)_n-$ where n is 2,3 or 4, $-CH_2CH(CH_3)-$ or $-CH_2C(CH_3)_2-$;
and their pharmaceutically acceptable salts.



Croydon Printing Company Ltd

86303225.6

0200524



# "DIHYDROPYRIDINE CALCIUM ANTAGONISTS"

## DESCRIPTION

This invention relates to certain dihydropyridines, specifically to certain 1,4-dihydropyridines having an amino-containing group attached to the 2-position, which have utility as anti-ischaemic and antihypertensive agents.

The compounds of the invention reduce the movement of calcium into the cell and they are thus able to delay or prevent the cardiac contracture which is believed to be caused by an accumulation of intracellular calcium under ischaemic conditions. Excessive calcium influx during ischaemia can have a number of additional adverse effects which would further compromise the ischaemic myocardium. These include less efficient use of oxygen for ATP production, activation of mitochondrial fatty acid oxidation and possibly, promotion of cell necrosis. Thus the compounds are useful in the treatment or prevention of a variety of cardiac conditions, such as angina pectoris, cardiac arrhythmias, heart attacks and cardiac hypertrophy. The compounds also have vasodilator activity since they can inhibit calcium influx in cells of vascular tissue and they are thus also useful as antihypertensive agents and for the treatment of coronary vasospasm.

In addition, because of their ability to inhibit calcium influx into smooth muscle cells, it is expected that they will have activity in the treatment of congestive heart failure, gastrointestinal disorders, cerebrovascular disorders, peripheral vascular disease, bronchopulmonary disorders, pulmonary hypertension, smooth muscle disorders of the urino-genital system, and hypertrophic cardiac myopathy.

According to the invention, there are provided 1,4-dihydropyridine derivatives of the formula:-

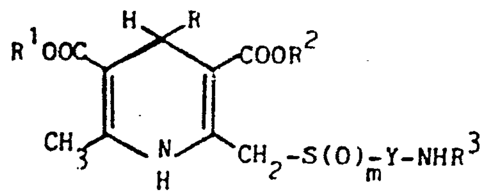

---- (I)

where R is an optionally substituted aryl or heteroaryl group;

$R^1$ and $R^2$ are each independently selected from

i) $C_1$-$C_4$ alkyl and

ii) $C_1$-$C_4$ alkyl substituted by $C_3$-$C_7$ cycloalkyl, aryl, $CF_3$, $C_2$-$C_4$ alkanoyl, halo, hydroxy, $C_1$-$C_4$ alkoxy, $C_2$-$C_4$ alkanoyloxy, aryl-($C_1$-$C_4$ alkoxy), or $-NR^4R^5$;

m is 0, 1 or 2;

$R^3$ is H, $C_1$-$C_4$ alkyl or $-COCF_3$;

$R^4$ is H or $C_1$-$C_4$ alkyl;

$R^5$ is H, $C_1$-$C_4$ alkyl or aryl-($C_1$-$C_4$ alkyl);

and Y is $-(CH_2)_n-$ where n is 2,3 or 4, $-CH_2CH(CH_3)-$ or $-CH_2C(CH_3)_2-$;

and their pharmaceutically acceptable salts.

The term "aryl" as used in this specification includes unsubstituted phenyl and phenyl substituted by, for example, one or two substituents each independently selected from nitro, halo, $C_1$-$C_4$ alkyl, $C_1$-$C_4$ alkoxy, hydroxy, trifluoromethyl, trifluoromethoxy, difluoromethoxy and cyano. It also includes 1- and 2-naphthyl.

"Halo" means F, Cl, Br or I.

The term "heteroaryl" as used in this specification for R means an aromatic heterocyclic group which may optionally be substituted and includes, for example, benzofuranyl; benzothienyl; pyridyl optionally monosubstituted by methyl, methylthio, halo or cyano; quinolyl; benzoxazolyl; benzothiazolyl; furyl; pyrimidinyl; thiazolyl; 2,1,3-benzoxadiazol-4-yl; 2,1,3-benzothiadiazol-4-yl; and thienyl optionally monosubstituted by halo or $C_1$-$C_4$ alkyl.

Alkyl and alkoxy groups having 3 or more carbon atoms can be straight or branched chain.

R is preferably phenyl substituted by 1 or 2 substituents each selected from halo (especially Cl) and $CF_3$, or is 2-chloropyrid-3-yl. Examples of R are 2-chlorophenyl, 2,3-dichlorophenyl, 2-chloro-3-trifluoromethylphenyl and 2-chloropyrid-3-yl. R is most preferably 2-chlorophenyl or 2,3-dichlorophenyl.

Preferably $R^1$ and $R^2$ are each independently $CH_3$ or $C_2H_5$.

Y is preferably $-(CH_2)_2-$ or $-(CH_2)_3-$.

The compounds of the formula (I) containing one or more asymmetric centres will exist as one or more pairs of enantiomers, and such pairs or individual isomers may be separable by physical methods, e.g. by fractional crystallisation or chromatography of the parent compounds or suitable derivatives thereof as will be known to those skilled in the art. The invention includes the separated pairs as well as mixtures thereof, as racemic mixtures or as separated optically-active isomeric forms.

The pharmaceutically acceptable acid addition salts of the compounds of the formula (I) are those formed from acids which form non-toxic acid addition salts containing pharmaceutically acceptable anions, such as the hydrochloride, hydrobromide, sulphate, phosphate or acid phosphate, acetate, maleate, fumarate, lactate, tartrate, citrate, methanesulphonate and gluconate salts.

The ability of the compounds of the formula (I) to inhibit the movement of calcium into the cell is shown by their effectiveness in reducing the contraction of

vascular tissue *in vitro* which is the consequence of calcium influx caused by a high extracellular concentration of potassium ions. The test is performed by mounting spirally cut strips of rat aorta with one end fixed and the other attached to a force transducer. The tissue is immersed in a bath of physiological saline solution containing 2.5 mM $Ca^{2+}$ and 5.9 mM $K^{\oplus}$. Potassium chloride is added to the bath with a pipette to give a final $K^{\oplus}$ concentration of 45 millimolar. The change in tension caused by the resulting contraction of the tissue is noted. The bath is drained and replaced with fresh saline solution and, after 45 minutes, the test is repeated with the particular compound under test present in the saline solution. The concentration of compound required to reduce the response by 50% ($IC_{50}$) is recorded.

The antihypertensive activity of the compounds is evaluated after oral administration by measuring the fall in blood pressure in spontaneously hypertensive rats or renally hypertensive dogs.

For administration to man in the curative or prophylactic treatment of cardiac conditions and hypertension, oral dosages of the compounds will be in the range of from 5-100 mg daily for an average adult patient (70 kg), typically 10-60 mg daily. Thus for a typical adult patient, individual tablets or capsules will generally contain 5, 10 or 20 mg of active compound, in a suitable pharmaceutically acceptable vehicle or carrier.

Dosages for intravenous administration will typically be within the range 1 to 10 mg per single dose as required. In practice the physician will determine the actual dosage which will be most suitable for an individual patient and it will vary with the age, weight and response of the particular patient. The above dosages are exemplary of the average case but there can, of course, be individual instances where higher or lower dosage ranges are merited, and such are within the scope of this invention.

For human use, the compounds of the formula (I) can be administered alone, but will generally be administered in admixture with a pharmaceutical carrier selected with regard to the intended route of administration and standard pharmaceutical practice. For example, they may be administered orally in the form of tablets containing such excipients as starch or lactose, or in capsules or ovules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other substances, for example, enough salts or glucose to make the solution isotonic.

Thus in a further aspect the invention provides a pharmaceutical composition comprising a compound of the formula (I) or a pharmaceutically acceptable salt thereof, together with a pharmaceutically acceptable diluent or carrier.

The invention also includes a compound of the formula (I) or or a pharmaceutically acceptable salt thereof, for use in medicine, in particular in the treatment of ischaemic heart disease, angina, or hypertension in a human being.

The invention also provides a method of protecting the heart from the deleterious effects of ischaemia, which comprises administering an effective amount of a compound of the formula (I) or pharmaceutically acceptable salt thereof, or a pharmaceutical composition as defined above.

The invention also includes a method of treating hypertension which comprises administering an antihypertensive amount of a compound of the formula (I) or pharmaceutically acceptable salt thereof, or pharmaceutical composition as defined above.

The compounds of the formula (I) in which m is O and $R^3$ is H or $C_1$-$C_4$ alkyl can be prepared by the reaction of a compound of the formula:-

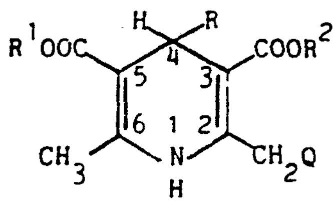

---- (II)

where R, $R^1$ and $R^2$ are as defined for formula (I) and Q is a leaving group, with a thiol (or salt thereof) of the formula:-

$R^3$-NH-Y-SH --- (III)

where $R^3$ is as defined in this method, and Y is as defined for formula (I).

When a salt of the thiol (III) is used the alkali metal salts, particularly the sodium and potassium salts, are preferred.

Q is a leaving group such as Cl, Br, tosyloxy, methanesulphonyloxy, trifluoromethanesulphonyloxy or 1-imidazolylsulphonyloxy.

The reaction is typically carried out in an organic solvent such as dioxane, dimethylformamide or tetrahydrofuran and compound (III) is preferably used in alkali metal salt form, preparable by the reaction of the free thiol with an alkali metal base such as potassium carbonate. Generally the reaction will proceed at from 0°C to room temperature but, if necessary, the reaction mixture can be heated to, say, 80°C, to accelerate the rate of reaction. If compound (III) is added in acid addition salt form, excess base (e.g. $K_2CO_3$) should be present.

The compounds of the formulae (II) and (III) are in general known compounds and, if not available commercially, can be obtained by conventional methods. For example, the compounds of the formula (II) are obtainable via the corresponding 2-methyl or 2-hydroxymethyl derivatives. Q is preferably Br or 1-imidazolylsulphonyloxy, and these starting materials are typically available by the bromination of the corresponding 2-methyl compounds using, e.g., pyridinium bromide perbromide [see Synthesis, 617, (1984)], or by reaction of the corresponding 2-hydroxymethyl compounds with sulphuryl chloride and imidazole.

The compounds in which m is 0 and $R^3$ is $-COCF_3$ can be prepared by the acylation of the corresponding compounds in which $R^3$ is H with an appropriate acylating agent, e.g. a $C_1$-$C_4$ alkyl trifluoroacetate (preferably ethyl trifluoroacetate), trifluoroacetic anhydride or trifluoroacetyl chloride. The reaction is typically carried out in an organic solvent such as ethanol, and in the presence of an organic base such as triethylamine or pyridine. Generally the reaction will proceed at a satisfactory rate at a temperature of from 0°C to room temperature (25°C). The product can then be recovered conventionally.

All the compounds of the formula (I) in which m is 1 or 2, ie, the sulphinyl and sulphonyl compounds, can be prepared by the oxidation of the corresponding thio compounds, typically by using about one (sulphinyl) or two (sulphonyl) equivalents of a suitable oxidising agent such as meta-chloroperbenzoic acid (preferred) or sodium metaperiodate.

To prepare the sulphinyl and sulphonyl compounds in which $R^3$ is H or $C_1$-$C_4$ alkyl it is however preferred to protect the primary or secondary amino group prior to oxidation by conventional means, removing the protecting group after oxidation. The preferred protecting group is trifluoroacetyl, which can be subsequently removed by alkaline hydrolysis, e.g. by using aqueous sodium

hydroxide in dioxane. Use of an N-protecting group tends to facilitate the recovery of the sulphinyl and sulphonyl end products. Other conventional amino-protecting groups can be used in place of trifluoroacetyl, e.g. $C_2$-$C_4$ alkanoyl or $C_1$-$C_4$ alkoxycarbonyl, both these being removable by alkaline hydrolysis, or phthalimido ($R^3$=H only), removable by treatment with ethanolic or aqueous methylamine, or hydrazine hydrate, or an alkali metal hydroxide (e.g. KOH) followed by a mineral acid (e.g. HCl) - see e.g. EP-A-0089167.

Acid addition salts are obtainable conventionally, e.g. by reacting a solution of the free base in an organic solvent with a solution of the acid in an organic solvent, followed by recovering the acid addition salt by filtration or evaporation of the solution.

The following Examples, in which all temperatures are in °C, illustrate the invention:-

EXAMPLE 1

2-(2-Aminoethylthiomethyl)-3,5-bis(methoxycarbonyl)-4-(2-chlorophenyl)-6-methyl-1,4-dihydropyridine hemifumarate

Pyridinium bromide perbromide (2.00g) was added in one portion to a stirred, ice-cooled solution of 3,5-bis(methoxycarbonyl)-4-(2-chlorophenyl)-2,6-dimethyl-1,4-dihydropyridine (1.68g) (See DT-OS 2302866 [1973]) and pyridine (0.80ml) in chloroform (50ml). The mixture was stirred at 0°C for 30 minutes, washed twice with ice-cold 0.25 M hydrochloric acid and once with water, dried over $Na_2SO_4$ and evaporated. A solution of the resulting oil in dioxane (10ml) was added to a mixture of potassium carbonate (5.54g) and 2-aminoethanethiol hydrochloride (2.26g) in a mixture of dioxane (50ml) and N,N-dimethylformamide (20ml) and the mixture was stirred at room temperature for 20 hours and evaporated. The residue was partitioned between water and ethyl acetate and the organic layer was washed with water, dried over $Na_2SO_4$, and evaporated. The residue was purified by chromatography on silica (8g) using dichloromethane plus 0-10% v/v methanol as the eluant. Appropriate fractions were combined and evaporated and the residue was taken up in ethanol and treated with an excess of a solution of fumaric acid in ether. The mixture was stirred overnight and the resulting solid was collected, washed with ether and dried in vacuo at 60-70° for four days to give the title compound, (0.32g), m.p. 180-185°.

Analysis % :-

| | |
|---|---|
| Found: | C,53.87; H,5.51; N,6.13 |
| $C_{20}H_{25}ClNO_4S$, 0.5 $C_4H_4O_4$ requires: | C,53.78; H,5.37; N,5.97. |

EXAMPLE 2

2-[3-Aminopropylthiomethyl]-3,5-bis(methoxycarbonyl)-4-(2-chlorophenyl)-6-methyl-1,4-dihydropyridine was prepared by the method described in Example 1 using 3-aminopropanethiol hydrochloride/$K_2CO_3$ instead of 2-aminoethanethiol hydrochloride/$K_2CO_3$. The product was characterised as the hemifumarate and had a m.p. of 150-160°.

Analysis % :-

| | |
|---|---|
| Found: | C,54.63; H,5.66; N,6.15; |
| $C_{20}H_{25}ClN_2O_4S$, 0.5 $C_4H_4O_4$ requires: | C,54.70; H,5.63; N,5.80. |

EXAMPLE 3

2-(2-Aminoethylthiomethyl)-3,5-bis(methoxycarbonyl)-4-(2,3-dichlorophenyl)-6-methyl-1,4-dihydropyridine was prepared by the method described in Example 1 but using 3,5-bis(methoxycarbonyl)-4-(2,3-dichlorophenyl)-2,6-dimethyl-1,4-dihydropyridine instead of 3,5-bis-(methoxycarbonyl)-4-(2-chlorophenyl)-2,6-dimethyl-1,4-dihydropyridine. The product was isolated as an oil which was not characterised analytically.

EXAMPLE 4

2-(2-Aminoethylthiomethyl)-4-(2-chlorophenyl)-3-ethoxycarbonyl-5-methoxycarbonyl-6-methyl-1,4-dihydropyridine

Sulphuryl chloride (1.08 g) was added dropwise to stirred, ice-cooled N,N-dimethylformamide (10 ml) over 10 minutes and the resulting solution was then added slowly to a stirred, ice-cooled solution of 4-(2-chlorophenyl)-3-ethoxycarbonyl-2-hydroxymethyl- 5-methoxycarbonyl-6-methyl-1,4-dihydropyridine (1.45 g) and imidazole (1.36 g) in N,N-dimethylformamide (20 ml). The mixture was stirred at 0°C for 20 minutes and at room temperature for 2 hours and then evaporated. The residue was partitioned between ethyl acetate and 2M hydrochloric acid and the layers separated. The organic layer was washed once with 2M hydrochloric acid and once with water, dried over sodium sulphate and evaporated. The residue was dissolved in tetrahydrofuran (10 ml) and the solution added to a stirred, ice-cooled mixture of 2-aminoethanethiol hydrochloride (1.13 g) and potassium carbonate (1.38 g) in tetrahydrofuran (20 ml). The mixture was stirred at 0°C for 2 hours and at room temperature for 8 days and then partitioned between ethyl acetate and water. The layers were separated and the organic layer washed with water, dried over sodium sulphate and evaporated. The residue was purified by chromatography on silica (14 g) using dichloromethane plus 0-25% ethyl acetate followed by dichloromethane plus 25% ethyl acetate plus 0-15% methanol as eluant. Appropriate fractions were combined and evaporated and the residue triturated with diethyl ether. The resulting solid was collected, washed with diethyl ether and dried to give the title compound (0.78 g), m.p. 116-119°C.

Analysis %:-

| | |
|---|---|
| Found: | C,56.69; H,6.18; N,6.44. |
| $C_{20}H_{25}ClN_2O_4S$ requires: | C,56.54; H,5.89; N,6.60. |

EXAMPLE 5

2-(2-Aminoethylthiomethyl)-4-(2,3-dichlorophenyl)-3-ethoxycarbonyl-5-methoxycarbonyl-6-methyl-1,4-dihydropyridine was prepared by the method described in the previous Example but using 4-(2,3-dichlorophenyl)-3-ethoxycarbonyl-2-hydroxymethyl-5-methoxycarbonyl-6-methyl-1,4-dihydropyridine instead of 4-(2-chlorophenyl)-3-ethoxycarbonyl-2-hydroxymethyl-5-methoxycarbonyl-6-methyl-1,4-dihydropyridine. The product was obtained as an essentially pure oil, which was not characterised analytically.

EXAMPLE 6

3,5-Bis(methoxycarbonyl)-4-(2-chlorophenyl)-6-methyl-2-[3-(trifluoroacetylamino)propylthiomethyl]-1,4-dihydropyridine hemihydrate

A solution of 2-(3-aminopropylthiomethyl)-3,5-bis(methoxycarbonyl)-4-(2-chlorophenyl)-6-methyl-1,4-dihydropyridine (4.40 g - see Example 2), ethyl trifluoroacetate (2.90 g) and triethylamine (3.0 g) in ethanol (50 ml) was stirred at room temperature for 2.5 hours and evaporated. The residue was purified by chromatography on $SiO_2$ (18 g) using hexane plus 50-100% dichloromethane as eluant. Appropriate fractions were combined and evaporated to give the title compound (3.0 g), as a hemihydrate, m.p. 50-60°.

Analysis %:-

| | |
|---|---|
| Found: | C,49.60; H,4.60; N,5.26; |
| $C_{22}H_{24}ClF_3N_2O_5S.0.5\ H_2O$ requires: | C,49.85; H,4.75; N,5.29. |

EXAMPLE 7

3,5-Bis(methoxycarbonyl)-4-(2-chlorophenyl)-6-methyl-2-[3-(trifluoroacetylamino)propylsulphinylmethyl]-1,4-dihydropyridine

A solution of 3,5-bis(methoxycarbonyl)-4-(2-chlorophenyl)-6-methyl-2-[3-(trifluoroacetylamino)-propylthiomethyl]-1,4-dihydropyridine (0.90 g - see Example 6) and meta-chloroperbenzoic acid (0.30 g) in a mixture of dichloromethane (40 ml) and 2-propanol (8 ml) was stirred at room temperature for 3 hours, diluted with dichloromethane, washed three times with 5% aqueous sodium hydrogen carbonate solution and once with brine, dried over $MgSO_4$ and evaporated. The residue was purified by chromatography on silica (4 g) using dichloromethane plus 0-3% methanol as eluant. Appropriate fractions were combined and evaporated to give the title compound (0.66 g), m.p. 85-90°.

Analysis %:-

| | |
|---|---|
| Found: | C,49.18; H,4.67; N,5.09; |
| $C_{22}H_{24}ClF_3N_2O_6S$ requires: | C,49.20; H,4.50; N,5.22. |

EXAMPLE 8

3,5-Bis(methoxycarbonyl)-4-(2-chlorophenyl)-6-methyl-2-[3-(trifluoroacetylamino)propylsulphonylmethyl]-1,4-dihydropyridine was prepared by the method described in the previous Example but using 3,5-bis-(methoxycarbonyl)-4-(2-chlorophenyl)-6-methyl-2-[3-(trifluoroacetylamino)propylsulphinylmethyl]-1,4-dihydropyridine instead of 3,5-bis(methoxycarbonyl)-4-(2-chlorophenyl)-6-methyl-2-[3-(trifluoroacetylamino)-propylthiomethyl]-1,4-dihydropyridine. The product had a m.p. of 75-80°.

Analysis %:-

| | |
|---|---|
| Found: | C,47.83; H,4.40; N,5.11; |
| Calculated for $C_{22}H_{24}ClF_3N_2O_7S$: | C,47.78; H,4.37; N,5.07. |

EXAMPLE 9

2-(3-Aminopropylsulphinylmethyl)-3,5-bis(methoxycarbonyl)-4-(2-chlorophenyl)-6-methyl-1,4-dihydropyridine hemihydrate

A solution of sodium hydroxide (90 mg) in water (2 ml) was added to a stirred, ice-cooled solution of 3,5-bis(methoxycarbonyl)-4-(2-chlorophenyl)-6-methyl-2-[3-trifluoroacetylamino)propylsulphinylmethyl]-1,4-dihydropyridine (0.60 g - see Example 7) in dioxane (20 ml). The mixture was stirred at 0° for 1.5 hours and at room temperature for 2 hours and evaporated. The residue was partitioned between ethyl acetate and water and the layers separated. The organic layer was washed twice with water, dried over $MgSO_4$ and evaporated to give the title compound (0.32 g) as a hemihydrate, m.p. 150°.

Analysis %:-

| | |
|---|---|
| Found: | C,53.55; H,5.94; N,5.91; |
| $C_{20}H_{25}ClN_2O_5S.0.5\ H_2O$ requires: | C,53.38; H,5.82; N,6.22. |

EXAMPLE 10

2-(3-Aminopropylsulphonylmethyl)-3,5-bis(methoxycarbonyl)-4-(2-chlorophenyl)-6-methyl-1,4-dihydropyridine was prepared by the method described in the previous Example but using 3,5-bis(methoxycarbonyl)-4-(2-chlorophenyl)-6-methyl-2-[3-(trifluoroacetylamino)-propylsulphonylmethyl]-1,4-dihydropyridine instead of 3,5-bis(methoxycarbonyl)-4-(2-chlorophenyl)-6-methyl-2-[3-(trifluoroacetylamino)propylsulphinylmethyl]-1,4-dihydropyridine. The product had a m.p. of 70-80°.

Analysis %:-

| | |
|---|---|
| Found: | C,52.69; H,5.68; N,5.81; |
| $C_{20}H_{25}ClN_2O_6S$ requires: | C,52.56; H,5.51; N,6.13. |

CLAIMS for the Contracting States:

BE, CH, DE, FR, GB, IT, LI, LU, NL, SE

1. A compound of the formula:-

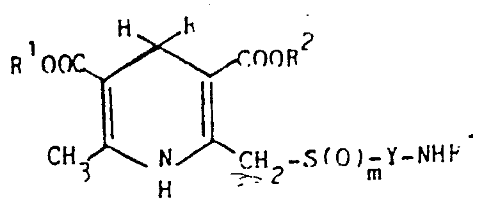

---- (I)

where R is an optionally substituted aryl or heteroaryl group;

$R^1$ and $R^2$ are each independently selected from

i) $C_1$-$C_4$ alkyl and

ii) $C_1$-$C_4$ alkyl substituted by $C_3$-$C_7$ cycloalkyl, aryl, $CF_3$, $C_2$-$C_4$ alkanoyl, halo, hydroxy, $C_1$-$C_4$ alkoxy, $C_2$-$C_4$ alkanoyloxy, aryl-($C_1$-$C_4$ alkoxy), or $-NR^4R^5$;

m is 0, 1 or 2;

$R^3$ is H, $C_1$-$C_4$ alkyl or $-COCF_3$;

$R^4$ is H or $C_1$-$C_4$ alkyl;

$R^5$ is H, $C_1$-$C_4$ alkyl or aryl-($C_1$-$C_4$ alkyl);

and Y is $-(CH_2)_n-$ where n is 2,3 or 4, $-CH_2CH(CH_3)-$ or $-CH_2C(CH_3)_2-$;

or a pharmaceutically acceptable salt thereof.

2. A compound as claimed in claim 1 wherein (a) "aryl" is either phenyl optionally substituted by one or two substituents each independently selected from nitro, halo, $C_1$-$C_4$ alkyl, $C_1$-$C_4$ alkoxy, hydroxy, trifluoromethyl, trifluoromethoxy, difluoromethoxy and cyano; or is 1- or 2-naphthyl; and (b) "heteroaryl" is benzofuranyl; benzothienyl; pyridyl optionally monosubstituted by methyl, methylthio, halo or cyano; quinolyl; benzoxazolyl; benzothiazolyl; furyl;

pyrimidinyl; thiazolyl; 2,1,3-benzoxadiazol-4-yl; 2,1,3-benzothiadiazol-4-yl; or thienyl optionally monosubstituted by halo or $C_1$-$C_4$ alkyl.

3. A compound as claimed in claim 2 wherein R is either phenyl substituted by 1 or 2 substituents each independently selected from halo and $CF_3$, or 2-chloropyrid-3-yl.

4. A compound as claimed in claim 3 wherein R is 2-chlorophenyl or 2,3-dichlorophenyl.

5. A compound as claimed in any one of the preceding claims wherein $R^1$ and $R^2$ are each independently $C_1$-$C_4$ alkyl.

6. A compound as claimed in claim 5 wherein $R^1$ and $R^2$ are each independently methyl or ethyl.

7. A compound as claimed in any one of the preceding claims wherein $R^3$ is H or $-COCF_3$.

8. A compound as claimed in any one of the preceding claims wherein Y is $-(CH_2)_n-$ where n is 2 or 3.

9. 2-(2-Aminoethylthiomethyl)-3,5-bis(methoxycarbonyl)-4-(2-chlorophenyl)-6-methyl-1,4-dihydropyridine or 2-(2-aminoethylthiomethyl)-4-(2-chlorophenyl)-3-ethoxycarbonyl-5-methoxycarbonyl-6-methyl-1,4-dihydropyridine.

10. A compound as claimed in claim 1 wherein $R^3$ is H or $C_1$-$C_4$ alkyl.

11. A pharmaceutical composition comprising a compound of the formula (I) as claimed in any one of the preceding claims, or a pharmaceutically acceptable salt thereof, and a pharmaceutically acceptable diluent or carrier.

12. A compound of the formula (I) as claimed in any one of claims 1 to 10, or a pharmaceutically acceptable salt thereof, for use as a medicament.

13. The use of a compound of the formula (I) as claimed in any one of claims 1 to 10, or a phramceutically acceptable salt thereof, in the manufacture of a medicament for use as an anti-ischaemic or antihypertensive agent.

CLAIMS for the Contracting State: AT

1. A process for preparing a compound of the formula:-

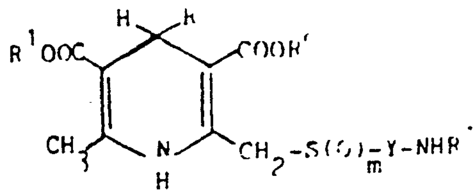

---- (I)

or a pharmaceutically acceptable salt thereof,

wherein R is an optionally substituted aryl or heteroaryl group;

$R^1$ and $R^2$ are each independently selected from

i) $C_1$-$C_4$ alkyl and

ii) $C_1$-$C_4$ alkyl substituted by $C_3$-$C_7$ cycloalkyl, aryl, $CF_3$, $C_2$-$C_4$ alkanoyl, halo, hydroxy, $C_1$-$C_4$ alkoxy, $C_2$-$C_4$ alkanoyloxy, aryl-($C_1$-$C_4$ alkoxy), or $-NR^4R^5$;

m is 0, 1 or 2;

$R^3$ is H, $C_1$-$C_4$ alkyl or $-COCF_3$;

$R^4$ is H or $C_1$-$C_4$ alkyl;

$R^5$ is H, $C_1$-$C_4$ alkyl or aryl-($C_1$-$C_4$ alkyl);

and Y is $-(CH_2)_n-$ where n is 2,3 or 4, $-CH_2CH(CH_3)-$ or $-CH_2C(CH_3)_2-$;

characterised by reacting a compound of the formula:-

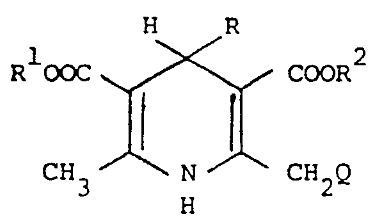

--- (II)

where R, $R^1$ and $R^2$ are as defined above and Q is a leaving group, with a thiol (or salt thereof) of the formula:-

$$R^3\text{-NH-Y-SH} \quad \text{--- (III)}$$

where $R^3$ is H or $C_1$-$C_4$ alkyl and Y is as defined above, thereby producing a compound of the formula (I) in which m is O and $R^3$ is H or $C_1$-$C_4$ alkyl;

said process being followed by, optionally, one or more of the following steps, as appropriate:-

i) acylation of a compound of the formula (I) in which $R^3$ is H so as to produce a compound of the formula (I) in which $R^3$ is $-COCF_3$;

ii) oxidation of a compound of the formula (I) in which m is O and $R^3$ is H, $C_1$-$C_4$ alkyl or $-COCF_3$ so as to produce a compound of the formula (I) in which m is 1 or 2;

iii) protecting the primary or secondary amino group of a compound of the formula (I) in which m is O and $R^3$ is H or $C_1$-$C_4$ alkyl, oxidising the resulting amino-protected compound so as to produce a compound in which m is 1 or 2, and then removing the protecting group; and

iv) conversion of a compound of the formula (I) into a pharmaceutically acceptable salt.

2. A process according to claim 1, characterised in that Q is Br or 1-imidazolylsulphonyloxy.

3. A process according to claim 1 or 2, characterised in that the reaction is carried out using an alkali metal salt of the thiol (III).

4. A process according to claim 3, characterised in that the salt is the potassium salt.

5. A process according to any one of the preceding claims, characterised in that (a) in optional step (i), the acylating agent has the formula:- $CF_3COO(C_1$-$C_4$ alkyl); (b) in optional step (ii), the oxidation is carried out with <u>meta</u>-chloroperbenzoic acid;

and (c) in optional step (iii), the primary or secondary amino group is protected with a trifluoroacetyl group by reaction with a $C_1$-$C_4$ alkyl trifluoroacetate, the oxidation is carried out with meta-chloroperbenzoic acid, and the trifluoroacetyl group is removed by alkaline hydrolysis.

6. A process for preparing a compound of the formula (I) as defined in claim 1 wherein R, $R^1$, $R^2$ and Y are as defined in claim 1, $R^3$ is H or $C_1$-$C_4$ alkyl, and m is 1 or 2, or a pharmaceutically acceptable salt thereof, which comprises oxidising a compound of the formula:-

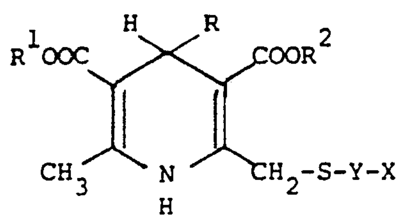

--- (IV)

where R, $R^1$, $R^2$ and Y are as defined in claim 1, and X is a protected primary or secondary amino group, said protected secondary amino group having the formula $-NR^3Z$ where $R^3$ is a $C_1$-$C_4$ alkyl group and Z is an amino-protecting group; said process being followed by removing the amino-protecting group and, optionally, by conversion of the product into a pharmaceutically acceptable salt.

7. A process according to claim 6, characterised in that the protected primary or secondary amino group has the formula $-NR^3.COCF_3$ where $R^3$ is H or $C_1$-$C_4$ alkyl, and in that the trifluoroacetyl protecting group is removed by alkaline hydrolysis.

8. A process according to any one of the preceding claims, characterised in that (a) "aryl" is either phenyl optionally substituted by one or two substituents each independently selected from nitro, halo, $C_1$-$C_4$ alkyl, $C_1$-$C_4$ alkoxy, hydroxy, trifluoromethyl, trifluoromethoxy, difluoromethoxy and cyano; or is 1- or 2-naphthyl; and (b) "heteroaryl" is benzofuranyl; benzothienyl; pyridyl

optionally monosubstituted by methyl, methylthio, halo or cyano; quinolyl; benzoxazolyl; benzothiazolyl; furyl; pyrimidinyl; thiazolyl; 2,1,3-benzoxadiazol-4-yl; 2,1,3-benzothiadiazol-4-yl; or thienyl optionally monosubstituted by halo or $C_1$-$C_4$ alkyl.

9. A process according to claim 8, characterised in that a compound in which (a) R is a phenyl group substituted by 1 or 2 substituents each independently selected from halo and $CF_3$; (b) $R^1$ and $R^2$ are each independently $C_1$-$C_4$ alkyl; (c) Y is $-(CH_2)_n-$ where n is 2 or 3 and (d) $R^3$ is H or $-COCF_3$, is prepared.

10. A process according to claim 9, characterised in that a compound in which (a) R is 2-chlorophenyl or 2,3-dichlorophenyl and (b) $R^1$ and $R^2$ are each independently methyl or ethyl, is prepared.

11. A process according to claim 1, characterised in that it is used to prepare a compound of the formula (I) as defined in claim 1, or a pharmaceutically acceptable salt thereof, in which R, $R^1$, $R^2$ and Y are as defined in claim 1, m is O, and $R^3$ is H or $C_1$-$C_4$ alkyl.